# EUROPEAN PATENT APPLICATION

(11) **EP 4 825 907 A1**
(43) Date of publication of application: **21.10.2026**
(21) Application number: 24902508.1
(22) Date of filing: 20.11.2024
(51) Int. Cl.: C08L 5/02, C12N 5/0775, C12N 5/071, C08L 67/08, C08L 91/00, C08J 3/24, C08J 3/12

(54) **MICROCARRIER, PREPARATION METHOD THEREFOR AND USE THEREOF, AND METHOD FOR CELL ADHERENT CULTURE AND RECOVERY**

(30) Priority: 11.12.2023 CN 202311689312
(71) Applicant: Wenzhou Institute, University of Chinese Academy Of Sciences, Wenzhou, Zhejiang 325000 (CN)
(72) Inventor: CHEN, Shangwu, Wenzhou, Zhejiang 325000 (CN); ZHENG, Jing, Wenzhou, Zhejiang 325000 (CN); XIA, Yulong, Wenzhou, Zhejiang 325000 (CN); WU, Yabin, Wenzhou, Zhejiang 325000 (CN)
(74) Representative: Winter, Brandl - Partnerschaft mbB
(86) International application number: PCT/CN2024/133086
(87) International publication number: WO 2025/124086

(57) **Abstract**

A microcarrier, a preparation method therefor and the use thereof, and a method for cell adherent culture and harvest. The prepared microcarrier can be used for cell culture, can maintain the survival rate of cells and can be quickly and completely dissolved during cell culture, and has a modified surface. Further provided in the present invention are the preparation of the microcarrier and the use of the microcarrier in cell adherent culture. Compared with an existing microcarrier, by means of using the microcarrier and method of the present application, the cell adherent culture can have a cell harvest efficiency of 95% or higher. In addition, by means of using a polysaccharide raw material, the introduction of an animal-derived pathogen into a cell product or a cell-derived biological product is prevented, and the manufacturing cost is significantly lower than that of raw materials such as recombinant gelatin and recombinant collagen.

## Description

### TECHNICAL FIELD

The present invention relates to the technical field of biomaterial, specifically to a microcarrier, preparation method therefor and use thereof, and a method for cell adherent culture and harvest.

### BACKGROUND ART

Cell culture is divided into two main categories: adherent culture, also known as monolayer culture or planar culture, and suspension culture. Cell culture can be used to produce cell therapy products such as stem cells, immune cells, and genetically modified cells for treating diseases, as well as cell-derived biological products such as viral vectors, vaccines, antibodies, peptide drugs, exosomes, and microvesicles.

Many cells derived from species such as humans, mammals, and insects need to adhere to the surface of materials in order to grow and proliferate. These cells are called adherent cells. Containers such as tissue culture dishes, tissue culture flasks and cell factories allow adherent cells to attach to the surface of flat materials such as polystyrene and to divide and proliferate. However, these culture containers can only provide a very limited growth surface for adherent cells, limiting the number of cells after expansion culture. In addition, microcarriers prepared from natural and /or synthetic polymers can provide a growth surface for adherent cells. Microcarriers used for cell culture are generally microspheres or quasi-spherical microparticles with a diameter of 100-300 micrometers. They can be solid or have a porous structure.

Christian Weber reported that after the cell lines of human mesenchymal stem cells were cultured with various microcarriers, Trypsin, Accutase, Collagenase or their mixed solutions were used to digest and harvest the cells. The results showed that the efficiency of cell harvest from the dextran microcarriers Cytodex 1 and Cytodex 3 was extremely low, with only 5%-25% of cells being harvested from the microcarrier surface. It is also found that it is difficult to separate mesenchymal stem cells using these proteases, and even with extended enzyme treatment time, the cell harvest rate was less than 50%.

### SUMMARY

In summary, although various microcarriers currently on the market can support the expansion of adherent cells, they have one or more of the following problems. (1) The efficiency of cell harvest from microcarriers after cell expansion is low; (2) The residual substances in microcarriers and animal-derived substances in microcarriers increase the safety risks of cell products or biological products produced by cells; (3) The large-scale use of high-priced raw materials such as recombinant proteins or peptides makes the manufacturing cost of microcarriers very high.

To address the shortcomings and deficiencies of existing technologies, the present invention provides a microcarrier, preparation method therefor and use thereof, as well as a method for cell adherent culture and harvest, which promotes adherent cell expansion and efficient harvest, is free of animal-derived substances and has low production costs.

The present invention provided a microcarrier, the microcarrier is a crosslinked microparticle obtained by crosslinking polysaccharide molecules, the microcarrier is able to maintain a stability of a structure of a polysaccharide microcarrier in a cell culture environment, and the microcarrier is able to be completely dissolved by enzyme.

In a preferred example of the present invention, the microcarrier is completely dissolved by enzyme within 2 hours.

In a preferred example of the present invention, the degree of crosslinking of polysaccharide molecules in the microcarrier is 13.1%-44.2%.

In another preferred example of the present invention, the crosslinked microparticles are crosslinked microspheres or quasi-spherical crosslinked microparticles.

In another preferred example of the present invention, the microcarrier is able to maintain the stability of a microsphere or quasi-spherical structure of the polysaccharide microcarrier for more than three days in a normal cell culture environment.

In another preferred example of the present invention, the microcarrier is able to maintain the stability of the microsphere or quasi-spherical structure of the polysaccharide microcarrier for more than three days in a cell culture environment of 25-41 °C.

In another preferred example of the present invention, the degree of crosslinking of polysaccharide molecules in microcarrier enables microcarrier to maintain the structure of polysaccharide microcarrier in cell culture environment and can be completely dissolved by enzyme, and the degree of crosslinking is a proportion of a number of monosaccharide residues that crosslink with a crosslinking agent or monosaccharide residues to a number of all monosaccharide residues that make up polysaccharide.

In another preferred example of the present invention, the microcarrier can be completely dissolved by enzyme within 30 minutes.

In another preferred example of the present invention, the degree of crosslinking of polysaccharide molecules in the microcarrier is 13.1%-38.7%.

In another preferred example of the present invention, the cell culture environment is capable of maintaining a cell survival rate of over 90%.

In another preferred example of the present invention, the microcarrier can be completely dissolved by an enzyme under conditions of 10-39 °C and pH 6.5-8.0.

In another preferred example of the present invention, the enzyme is a polysaccharide enzyme.

In another preferred example of the present invention, the polysaccharide enzyme includes one or more of amylase, cellulase, glucanase, alginase, chitosanase, hyaluronidase, agarase, and pectinase.

In another preferred example of the present invention, the surface of the microcarrier is smooth or porous.

In another preferred example of the present invention, the pore size of the porous structure is greater than 1µm and less than 100µm.

In another preferred example of the present invention, conditions for controlling the pore size of the porous structure comprise freezing temperature and freezing rate.

In another preferred example of the present invention, the surface of the microcarrier is modified.

In another preferred example of the present invention, the degree of crosslinking of polysaccharide molecules in the surface-modified microcarrier is 13.6-44.3%.

In another preferred example of the present invention, a surface modification of the microcarrier is one or more of positively charged groups or molecules bonded by chemical bonds on the surface, or positively charged groups or molecules physically adsorbed, or proteins, polypeptides or polysaccharides promoting cell adhesion through chemical bonding or physical adsorption.

In another preferred example of the present invention, the positively charged groups or molecules bonded by chemical bonds on the surface include, but are not limited to, primary amines, secondary amines, tertiary amines, and quaternary ammonium salts.

In another preferred example of the present invention, the physically adsorbed positively charged groups or molecules comprise cationic polymers in diethylaminoethyl dextran, poly -L-ornithine (PLO), poly -D- ornithine (PDO), poly -DL- ornithine, poly -D- lysine (PDL), poly -L-lysine (PLL), poly- DL- lysine, poly -L- arginine (PLA), poly -D- arginine (PDA), poly -DL - arginine, poly -L- homoarginine (PLHA), poly -D- homoarginine (PDHA), poly -DL-homoarginine, poly -L- histidine (PLH), poly -D -histidine (PDH), poly -DL-histidine, polymethylene -CO- guanidine (PMCG), polyallylamine (PAA), and polyvinylamine (PVA), polyethyleneimine, allylamine-diallylamine copolymer and allylamine-maleic acid copolymer.

In another preferred example of the present invention, the proteins or polypeptides promoting cell adhesion comprises collagen, gelatin, laminin, fibronectin, vitronectin, fragments of collagen, gelatin, laminin, fibronectin or vitronectin promoting cell adhesion, and polypeptides containing arginylglycylaspartic acid.

In another preferred example of the present invention, the polysaccharide comprises one or more of following substances: starch, cellulose, dextran, sodium alginate, chitosan, hyaluronic acid, agarose, polygalacturonic acid and pectin, and derivatives of starch, cellulose, dextran, sodium alginate, chitosan, hyaluronic acid, agarose, polygalacturonic acid and pectin derivatives.

In another preferred example of the present invention, the crosslinking method includes one or more of the following methods: chemical crosslinking, photo crosslinking, dehydrogenation thermal crosslinking, and ionic crosslinking.

In another preferred example of the present invention, the crosslinking agent used for crosslinking or fixing a chain structure of the polysaccharide molecules in the chemical crosslinking comprise but are not limited to: 1,3- dichloropropanol, N,N' -methylenebisacrylamide, 2,3- dibromo -1- propanol, 1,2,7,8- diepoxyoctane, divinyl sulfone, glutaraldehyde, 1,4-butanediol diglycidyl ether, 1- ethyl- (3- dimethylaminopropyl) carbodiimide hydrochloride, N-hydroxy succinimide, epichlorohydrin, tannic acid, or genipin.

In another preferred example of the present invention, the photo crosslinking is carried out by a light source that is able to initiate a reaction between the crosslinking agent and the polysaccharide molecules.

In another preferred example of the present invention, the light source is ultraviolet light.

In another preferred example of the present invention, the dehydrogenation thermal crosslinking is carried out in a near-vacuum environment and at high temperature.

The present invention also provides a use of microcarrier in the culture of adherent cells.

In a preferred embodiment of the present invention, the cultured cells include, but are not limited to, insect cells, mammalian cells, bird cells, and human cells.

In another preferred example of the present invention, the culture temperature of mammalian cells or human cells is 36-38 degrees Celsius, and the pH is 6.8-8.2.

In another preferred example of the present invention, the method comprises:
forming a water-in-oil emulsion with polysaccharide solution, organic solvent which is immiscible with water, and emulsifier liquid, adding a crosslinking agent to make polysaccharide undergoing a crosslink reaction;
or, adding a crosslinking agent to polysaccharide solution at first to make polysaccharide undergoing a crosslink reaction, and then forming a water-in-oil emulsion with the crosslinked polysaccharide solution, organic solvent which is immiscible with water, and emulsifier liquid.

In another preferred example of the present invention, the method for forming the water-in-oil emulsion includes, but is not limited to, mechanical stirring, homogenization emulsification, membrane emulsification, spraying, ultrasonic emulsification, ultrasonic spraying, and microfluidic method.

In another preferred example of the present invention, the method further includes washing to remove impurities other than organic solvents and microcarrier.

In another preferred example of the present invention, the concentration of polysaccharides in the polysaccharide solution is 10%-50% w/w.

In another preferred example of the present invention, the method further comprises adding a positively charged substance or a substance promoting cell adhesion after adding the crosslinking agent or at the same time as adding the crosslinking agent, so that a surface of a polysaccharide microspheres is positively charged or contains cell adhesion molecules through physical adsorption or chemical reaction.

In another preferred example of the present invention, the organic solvent which is immiscible with water is vegetable oil.

In another preferred example of the present invention, the emulsifier comprises, but is not limited to, lecithin, carrageenan, guar gum, xanthan gum, polysorbate, cellulose, fatty acid monoglycerides and diglycerides, sucrose esters and sucrose glycerides, fatty acid polyglycerides, polyglycerol polyricinoleate, stearoyl lactate, and dehydrated sorbitol ester.

In another preferred example of the present invention, the polyglycerol polyricinoleate has a volume fraction of 1-10% in the organic solvent.

In another preferred example of the present invention, the crosslinking agent is epichlorohydrin.

In another preferred example of the present invention, the molar ratio of epichlorohydrin to monosaccharide residues in the polysaccharide is 0.23-0.53:1.

In another preferred example of the present invention, the crosslinking reaction takes more than 20 hours.

The present invention also provides a use of the microcarrier as a material for adherent cell culture.

The present invention also provides a method for cell adherent culture and cell harvest: mixing the microcarrier, culture medium and adherent cells, performing cell culture, then washing the microcarrier, simultaneously or stepwise adding polysaccharide enzyme and enzyme that decompose proteins or peptides to dissolve the microcarrier, confirming that the microcarrier is completely dissolved, and harvesting the adherent cells.

The present invention also provides a method for dissolving microcarrier. For microcarrier whose surface is modified with proteins or peptides promoting cell adhesion, polysaccharide enzyme and enzyme that decomposes proteins or polypeptides are used simultaneously or stepwise to dissolve microcarrier.

The present invention has the beneficial effects that the invention provides a microcarrier, a preparation method therefor and the use thereof, and a method for cell adherent culture and harvest. The prepared microcarrier can be completely dissolved. Compared with an existing microcarrier, by means of using the microcarrier and method of the present application, the cell adherent culture can have a cell harvest efficiency of 95% or higher. In addition, by means of using a polysaccharide raw material, the introduction of an animal-derived pathogen into a cell product or a cell-derived biological product is prevented, and the manufacturing cost is significantly lower than that of raw materials such as recombinant gelatin and recombinant collagen.

### BRIEF DESCRIPTION OF THE DRAWINGS

FIG. 1 shows various microcarriers before and half an hour after enzyme treatment.
FIG. 2 shows cell attachment and staining for live and dead cells (left: phase contrast microscopy image; middle: live cells stained white; right: dead cells stained black).
FIG. 3 shows cell expansion using microcarriers.
FIG. 4 shows the microcarriers and cells before (left) and after (right) enzyme treatment.
FIG. 5 shows the staining of cells attached to various microcarriers (left), live cells (middle), and dead cells (right).
FIG. 6 shows positively charged modified microcarriers and cells before (left) and after (right) enzyme treatment.
FIG. 7 shows a microcarrier with a porous surface.

### DETAILED DESCRIPTION

The present invention will be further described below with reference to the accompanying drawings and the following embodiments. It should be understood that the accompanying drawings and the following embodiments are for illustrative purposes only and are not intended to limit the present invention.

Polysaccharides can be extracted from plants or produced by microbial fermentation. They are widely available, have low production costs, and good biocompatibility, making them suitable for large-scale preparation of cell culture microcarriers. The hydroxyl, amino, and carboxyl groups of polysaccharide molecules can be used to fix the polysaccharide molecules in polysaccharide microspheres and prevent the dissolution of polysaccharide microcarriers during cell culture by using chemical crosslinkers or physical crosslinking methods. By controlling the type, content, reaction temperature, and reaction time of the chemical crosslinking agent in the crosslinking reaction, the degree of crosslinking of polysaccharides in the microcarriers can be controlled. This not only maintains the structure of the polysaccharide microcarriers in the cell culture environment, but also allows the microcarriers to be completely dissolved by polysaccharide enzymes after cell expansion culture, thus achieving efficient cell harvest.

Polysaccharides suitable for preparing microcarriers include starch, cellulose, dextran, sodium alginate, chitosan, hyaluronic acid, agarose, polygalacturonic acid, and pectin and its derivatives.

In order to prepare microcarriers, it is necessary to form droplets of polysaccharide solution to control the size and shape of microcarriers. The method for forming the polysaccharide solution droplets or water-in-oil emulsion includes, but is not limited to, mechanical stirring, homogenization emulsification, membrane emulsification, spraying, ultrasonic emulsification, ultrasonic spraying, and microfluidic method. In emulsification, droplets of a polysaccharide solution need to be formed in an organic solvent which is immiscible with water. The organic solvent which is immiscible with water include, but are not limited to toluene, mineral oil, paraffin oil, vegetable oil including rapeseed oil, corn oil, cottonseed oil, safflower oil, soybean oil, extra virgin olive oil, sunflower oil, palm oil, MCT oil, and trioleic acid oil. In order to maintain the stability of the emulsion obtained after emulsification, it is necessary to add emulsifiers in the emulsification process, including but not limited to, lecithin, carrageenan, guar gum, xanthan gum, polysorbate, cellulose (including carboxymethyl cellulose), fatty acid monoglycerides and diglycerides, sucrose esters and sucrose glycerides, fatty acid polyglycerides, polyglycerol polyricinoleate, stearoyl lactate, and dehydrated sorbitol ester.

Methods for crosslinking polysaccharides include chemical crosslinking, photo crosslinking, dehydrothermal crosslinking, and ionic crosslinking and so on. Chemical crosslinking methods utilize crosslinking agents such as glutaraldehyde, 1,4-butanediol diglycidyl ether, 1- ethyl- (3- dimethylaminopropyl) carbodiimide hydrochloride (EDC), N-hydroxysuccinimide (NHS), epichlorohydrin, tannic acid, and genipin to crosslink or fix the chain structure of polysaccharide molecules. Photo crosslinking can be initiated by light sources such as ultraviolet light to cause a reaction between the crosslinking agent and polysaccharide molecules. Dehydrogenation thermal crosslinking refers to the process of using a near-vacuum environment and high temperatures to react the carboxyl, hydroxyl, and amino groups on polymers, thereby crosslinking the polymers.

The degree of crosslinking of polysaccharides in microcarrier can be defined as proportion of a number of monosaccharide residues that crosslink with a crosslinking agent or functional groups of monosaccharide residues among monosaccharide residues that make up polysaccharide to a number of all monosaccharide residues. The functional groups such as hydroxyl and carboxyl groups of the monosaccharide residues that make up polysaccharides can form chemical bonds with crosslinking agents, and the functional groups of the monosaccharide residues that make up polysaccharides can also form chemical bonds with each other under certain conditions. If the monosaccharide residues that make up polysaccharides do not undergo a chemical reaction, they can be oxidized by sodium periodate to form formic acid. By determining the amount of formic acid formed after the oxidation reaction through acid-base titration, the amount of monosaccharide residues that did not undergo chemical reaction can be determined, thereby calculating the amount of monosaccharide residues that did undergo chemical reaction and the degree of crosslinking of polysaccharides. Assuming the mass of the polysaccharide is m (unit: g), and the formic acid produced after oxidation is n (unit: mol), the number of monosaccharide residues that did not undergo chemical reaction can be calculated to be n (unit: mol). The proportion of these monosaccharide residues to all monosaccharide residues (with a molecular weight of A) in the polysaccharide is n*A/m . The proportion of monosaccharide residues that underwent chemical reaction can be calculated to be 1-n*A/m , which is the degree of polysaccharide crosslinking as defined here. In addition, methods for detecting the degree of polysaccharide crosslinking include, but are not limited to, nuclear magnetic resonance (NMR) and total ion chromatography (TIC).

To encourage cells to adhere to the surface of microcarriers, the surface of the microcarriers can be positively charged or contain proteins, peptides, or other molecules that promote cell adhesion. The positively charged groups include primary amine, secondary amine, tertiary amine and quaternary ammonium salt, which can be attached to the surface of microcarrier by chemical reaction. In addition, by adsorbing polymers with positively charged groups, for example cationic polymers in diethylaminoethyl dextran, poly -L- ornithine (PLO), poly -D-ornithine (PDO), poly -DL- ornithine, poly -D- lysine (PDL), poly -L- lysine (PLL), poly- DL-lysine, poly -L- arginine (PLA), poly -D- arginine (PDA), poly -DL -arginine, poly -L-homoarginine (PLHA), poly -D- homoarginine (PDHA), poly -DL- homoarginine, poly -L-histidine (PLH), poly -D -histidine (PDH), poly -DL-histidine, polymethylene -CO- guanidine (PMCG), polyallylamine (PAA), and polyvinylamine (PVA), polyethyleneimine, allylamine-diallylamine copolymer and allylamine-maleic acid copolymer, cells with negatively charged surfaces can also be attached to microcarrier. Proteins and peptides that promote cell adhesion can be obtained from animal tissues or through recombinant protein technology. They include, but are not limited to collagen, gelatin, laminin, fibronectin, vitronectin, or fragments of the aforementioned proteins that promote cell adhesion, as well as peptides containing arginylglycylaspartic acid (RGD).

The culture environment for adherent cells using microcarriers varies depending on the type of adherent cell. The suitable temperature for insect cell culture is generally 26-30 degrees Celsius, and the pH is 6.0-6.4. The suitable temperature for mammalian cell culture is generally 36-38 degrees Celsius, and the pH is 6.8-8.2. The suitable temperature for bird cell culture is generally 39-41 degrees Celsius, and the pH is 7.0-8.5. For example, the optimal temperature for culturing chicken satellite cells is 41 degrees Celsius. Chicken embryo cells can be passaged and cultured for a long time in an environment with a pH of 8.1-8.5. Insect cells grow slower in environments below 26 degrees Celsius, and their viability decreases in environments above 30 degrees Celsius. Cell viability is the proportion of live cells in a cell sample, which can be determined by staining dead and/or live cells, as well as by using a hemocytometer or automated cell counter.

Depending on the type of polysaccharide in the microcarrier, the polysaccharide enzyme that dissolves the microcarrier can be amylase, cellulase, glucanase, alginase, chitosanase, hyaluronidase, agarase, pectinase, or a combination thereof. If the surface of polysaccharide microcarriers is modified with proteins or polypeptides that promote cell adhesion, the microcarriers can be dissolved simultaneously or stepwise with enzymes that dissolve proteins or polypeptides, such as polysaccharide enzyme, collagenase, trypsin, papain and TrypLE enzyme. For example, the protein or polypeptide of the microcarrier is dissolved by an enzyme which decomposes the protein or polypeptide at first, and then the crosslinked polysaccharide is dissolved by a polysaccharide enzyme. Or the crosslinked polysaccharide is dissolved by polysaccharide enzyme at first, and then the enzyme that decomposes protein or polypeptide is used. The conditions for dissolving polysaccharide microcarriers with enzymes should maintain cell viability, for example, conditions such as temperature and pH should not affect cell viability. For example, when culturing human mesenchymal stem cells using microcarriers, the conditions for dissolving polysaccharide microcarriers can be 10-39 °C and pH 6.5-8. Cells are more likely to die if the temperature is above 39 °C, the pH is below 6.5, or the pH is above 8.

Microscopic observation can be used to detect whether polysaccharide microcarriers are completely dissolved. Complete dissolution here refers to the absence of spherical or quasi-spherical microcarriers under a conventional optical microscope, as well as the particulate matter remaining after partial dissolution of the microcarriers. A conventional optical microscope refers to an optical microscope that uses 4-20x objectives and 10x eyepieces for observation. For microcarrier samples without cultured cells, the microcarriers can be directly treated with polysaccharide enzymes. After dissolution, the presence of microcarrier residues can be observed using a conventional optical microscope. After culturing cells with microcarriers and dissolving the microcarriers with polysaccharide enzymes, the cells can be dissolved using cell lysis buffer, and then observed using a conventional optical microscope to see if any microcarrier residue remains.

The surface of a microcarrier can be smooth or porous. The smooth surface here refers to the surface of the microcarrier that does not have a clearly visible uneven structure when observed under a conventional optical microscope. This smooth surface allows adherent cells to extend onto the surface of the microcarrier and proliferate. The surface of the microcarrier can also be porous, allowing adherent cells to grow inside the pores. The pore size of the microcarrier surface can be 2-100µm, 10-80µm, or 15-50µm. The pore size of microcarriers can be controlled by factors such as freezing temperature and freezing rate during material preparation. Generally, within the range of 0 to liquid nitrogen temperature, the lower the freezing temperature, the smaller the pore size of the resulting microcarrier material.

### Example 1 Preparation of the microcarrier

Dextran was dissolved in 6 mL of 5 M sodium hydroxide solution to obtain a 40% (w/w) dextran solution. Polyglycerol polyricinoleate (PGPR) was dissolved in soybean oil to obtain oil phases of 0.5%, 1%, 2%, 3%, 5%, and 10% PGPR (v/v). Six portions of 1g dextran solution were taken, 1mL of each of the above six oil phase liquids was added respectively, and then 50µL of epichlorohydrin crosslinking agent was added. Each mixture was mixed at 1000 rpm for 3 minutes through a high-speed homogenizer respectively. The results after mixing showed that the oil phase containing 1-10% (v/v) PGPR could form a stable water-in-oil emulsion.

Dextran was dissolved in 6 mL of 5 M sodium hydroxide solution to obtain a 40% (w/w) dextran solution. According to Table 1, four portions of 1g dextran solutions were taken, 1mL soybean oil and 20µL polyglycerol polyricinoleate (PGPR) were added respectively, and then 100µL, 75µL, 50µL or 25µL epichlorohydrin crosslinking agent was added. Each of the four mixtures was mixed at 1000 rpm for 3 minutes through a high-speed homogenizer, and the resulting four emulsions were added to 2 ml centrifuge tubes respectively. Each centrifuge tube was placed in a 50-degree oven for crosslinking reaction, and taken out after 20 hours. The reacted emulsion was transferred to a 50mL centrifuge tube, 10mL of acetone was added and stirred and washed. After standing, the supernatant was removed and 10mL of anhydrous ethanol was added to wash. The crosslinked dextran microspheres were then washed six times with pure water. In particular, Sample 4 was partially dissolved during the washing process. 0.04 mL of 0.5 g/mL DEAE-dextran solution was added to the remaining three samples and mixed for 2 hours at room temperature using a rotary mixer to coat the surface of the dextran microspheres with DEAE-dextran molecules to obtain microcarriers. Then the microcarriers were washed 6 times with pure water and 2 times with phosphate buffered saline (PBS).

After shaking the microcarriers of samples 1-3 in PBS evenly, 0.1 mL was taken and added to a 96-well plate. The 96-well plate was placed in a cell culture incubator at 37 degrees Celsius for 3 days. It was observed with the 4x and 10x objectives of an inverted optical microscope that the microcarriers maintained their structural integrity and did not dissolve. Glucanase solution (1000 u/mL) was added to the microcarriers in the 96-well plate and mixed well. Then the 96-well plate was placed in a 37 °C cell culture incubator for 30 minutes. Then, the 96- well plate was taken out and observed using the 4x and 10x objectives of an inverted optical microscope. The results showed that only part of the microcarrier in sample 1 dissolved, while the microcarriers in samples 2 and 3 dissolved completely, and no residual microcarriers or particulate matter were observed.

**Table 1. Raw material composition and dissolution results of polysaccharide microcarrier after enzyme treatment**

| Sample number | Sample 1 | Sample 2 | Sample 3 | Sample 4 |
|---|---|---|---|---|
| Dextran solution (g) | 1 | 1 | 1 | 1 |
| Soybean oil (mL) | 1 | 1 | 1 | 1 |
| PGPR (µL) | 20 | 20 | 20 | 20 |
| crosslinking agent (µL) | 100 | 75 | 50 | 25 |
| Whether it can be dissolved by enzymes | It can be dissolved partially. | It can be dissolved completely. | It can be dissolved completely. | - |

### Example 2 Preparation of the microcarrier

Dextran was dissolved in 6 M sodium hydroxide solution to obtain a 30% (w/w) dextran solution. According to Table 2, three portions of 1 g dextran solution were taken, 50µL, 44µL or 37.5µL epichlorohydrin crosslinking agent was added respectively. Then 1mL of soybean oil and 20µL of polyglycerol polyricinoleate (PGPR) were added respectively, each of the three mixtures was mixed at 500 rpm for 3 minutes through a high-speed homogenizer, and the resulting three emulsions were added to 2 ml centrifuge tubes respectively. Each centrifuge tube was placed in a 50-degree oven for crosslinking reaction, and removed after 24 hours. The reacted emulsion was transferred to a 50mL centrifuge tube, 10mL of acetone was added and stirred and washed. After standing, the supernatant was removed and 10mL of anhydrous ethanol was added to wash. The crosslinked dextran microspheres were then washed six times with pure water. In particular, Sample 6 was partially dissolved during the washing process. 0.04 mL of 0.5 g/mL DEAE-dextran solution was added to the remaining two samples and mixed for 2 hours at room temperature using a rotary mixer to coat the surface of the dextran microspheres with DEAE- dextran to obtain microcarriers. Then the microcarriers were washed 6 times with pure water and 2 times with PBS.

After shaking the microcarriers of samples 4-5 evenly, 0.1 mL was taken and added to a 96-well plate. The 96-well plate was placed in a cell culture incubator at 37 degrees Celsius for 3 days. It was observed with the 4x and 10x objectives of an inverted optical microscope that the microcarriers maintained their structural integrity and did not dissolve. Glucanase solution (1000 u/mL) was added to the microcarriers in the 96-well plate and mixed well. Then the 96-well plate was placed in a 37 °C cell culture incubator for 30 minutes. Then, the 96- well plate was taken out and observed using the 4x and 10x objectives of an inverted optical microscope. The results showed that the microcarriers in samples 4 and 5 dissolved completely, and no residual microcarriers or particulate matter were observed.

**Table 2. Raw material composition and dissolution results of polysaccharide microcarrier after enzyme treatment.**

| Sample number | Sample 4 | Sample 5 | Sample 6 |
|---|---|---|---|
| Dextran solution (g) | 1 | 1 | 1 |
| Soybean oil (mL) | 1 | 1 | 1 |
| PGPR (µL) | 20 | 20 | 20 |
| crosslinking agent (µL) | 50 | 44 | 37.5 |
| Whether it can be dissolved by enzymes | It can be dissolved completely. | It can be dissolved completely. | - |

### Example 3 Preparation of the microcarrier

40g Dextran was dissolved in 60 mL of 5 M sodium hydroxide solution. According to Table 3, eight portions of 10g dextran solutions were taken, then 10mL soybean oil and 200µL polyglycerol polyricinoleate (PGPR) were added to each portion, and then 400µL, 500µL, 600µL, 700µL, 800µL, 900µL, 1200µL or 1500µL of epichlorohydrin crosslinking agent was added. The molar ratio of epichlorohydrin to monosaccharide residues in the polysaccharide is 0.23:1, 0.29:1, 0.35:1, 0.41:1, 0.47:1, 0.53:1, 0.70:1 and 0.88:1, respectively. Each of the eight mixtures was mixed at 200 rpm for 3 minutes through a mechanical mixer, and the resulting eight emulsions were added to 50 ml centrifuge tubes respectively. Each centrifuge tube was placed in a 50°C oven for crosslinking reaction, and removed after 24 hours. The reacted emulsion was transferred to a 500mL beaker, 50mL of acetone was added and stirred and washed. After standing, the supernatant was removed and 50mL of anhydrous ethanol was added to wash. Then it was washed with pure water for 6 times to obtain crosslinked dextran microspheres. 0.04 mL of 0.5 g/mL DEAE-dextran solution was added to each sample and mixed for 2 hours at room temperature using a rotary mixer to coat the surface of the dextran microspheres with DEAE- dextran to obtain microcarriers. Then the microcarriers were washed 6 times with pure water and 2 times with PBS.

After the eight microcarriers were shaken evenly, 0.1 mL of each of them was taken and added to a 96-well plate. The 96-well plate was placed in a cell culture incubator at 37 degrees Celsius for 3 days. It was observed with the 4x and 10x objectives of an inverted optical microscope that the microcarriers maintained their structural integrity and did not dissolve. Glucanase solution was added to the microcarriers in the 96-well plate and mixed well. Then the 96-well plate was placed in a 37 °C cell culture incubator for 30 minutes. Then, the 96-well plate was taken out and observed using the 4x and 10x objectives of an inverted optical microscope.

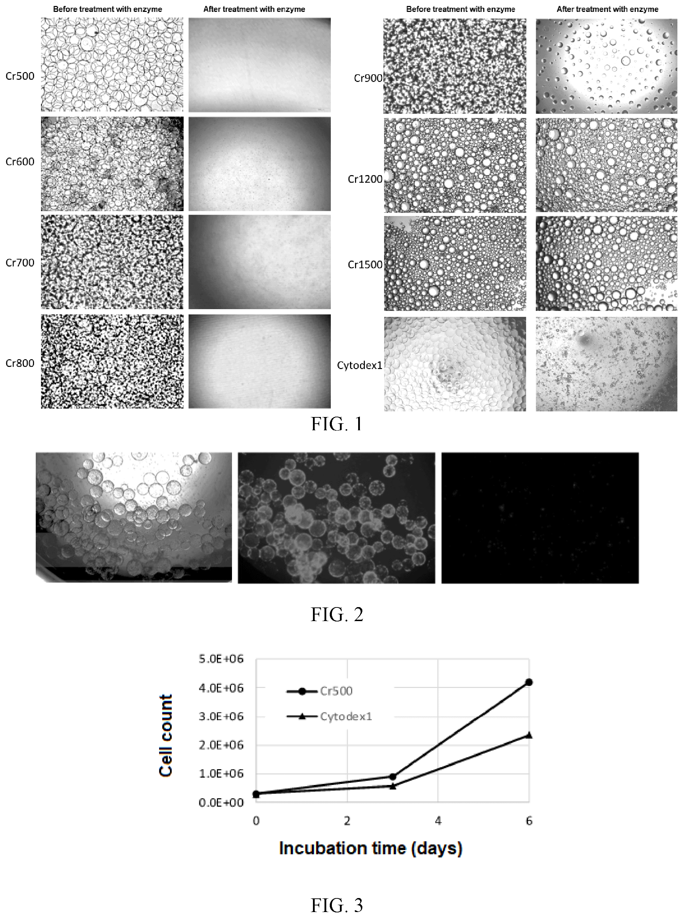

After 30 minutes of enzyme treatment, the results showed that the Cr400, Cr500, Cr600, Cr700, and Cr800 microcarrier samples could be completely dissolved by the enzyme, and no residual microcarriers or particles were observed. Only a portion of Cr900 dissolved, resulting in a reduction in the overall size of the microcarrier. The size and shape of the Cr1200 and Cr1500 microcarriers did not change significantly, remaining spherical or quasi-spherical (FIG. 1). The enzyme treatment time was extended to 2 hours, the Cr900 microcarriers gradually dissolved and disappeared, while the size and shape of the Cr1200 and Cr1500 microcarriers did not change significantly (Table 3). The sample that can be dissolved by enzymes within 2 hours corresponds to a molar ratio of epichlorohydrin to monosaccharide residues in the polysaccharide during the preparation process of 0.23 to 0.53:1.

Similarly, when the commercially available Cytodex 1 microcarriers (Cytiva) were treated with enzymes for 30 minutes, partial dissolution of the microcarriers was observed under an optical microscope, leaving many particulate residues ranging from a few micrometers to tens of micrometers, as well as a small number of spherical microcarriers (FIG. 1). When the enzyme treatment time was extended to 2 hours, the microcarrier residues could not be eliminated.

**Table 3. Raw material composition and dissolution results of polysaccharide microcarrier after enzyme treatment.**

| Sample name | Cr400 | Cr500 | Cr600 | Cr700 | Cr800 | Cr900 | Cr1200 | Cr1500 |
|---|---|---|---|---|---|---|---|---|
| Dextran solution (g) | 10 | 10 | 10 | 10 | 10 | 10 | 10 | 10 |
| Soybean oil (mL) | 10 | 10 | 10 | 10 | 10 | 10 | 10 | 10 |
| PGPR (µL) | 200 | 200 | 200 | 200 | 200 | 200 | 200 | 200 |
| Crosslinking agent (µL) | 400 | 500 | 600 | 700 | 800 | 900 | 1200 | 1500 |
| Whether it can be dissolved by enzymes | It can be dissolved completely. | It can be dissolved completely. | It can be dissolved completely. | It can be dissolved completely. | It can be dissolved completely. | It can be dissolved completely. | Insoluble | Insoluble |

### Example 4: Measurement of the degree of crosslinking of microcarrier

40 mL of each of the various microcarriers obtained in Example 3 was taken and placed in a 50 mL beaker. In addition, 0.3g of Cytodex 1 microcarrier and 0.3g of dextran were taken and placed into a 50mL beaker. The beakers containing different samples were placed in a 70°C forced-air dryer and dried for more than 24 hours until the sample weight change is less than 0.002g. Then, 0.200 g of each dried sample was taken and placed into a 100 mL glass bottle.

5.35g of sodium periodate was dissolved in 500mL of pure water. 50mL of sodium periodate solution was added to each glass bottle containing the dry sample. A magnetic stir bar was placed in the bottle. The bottle was covered with the cap and wrapped with aluminum foil to block light. Each reaction vessel was placed at room temperature (20 °C) and mixed and oxidized using magnetic stirring. The pH of the reaction solution was measured with a pH meter every 3-6 hours until the pH change is less than 0.02 over 3-6 hours, at which point the reaction was considered complete.

10 mL of the supernatant from each 100 mL glass bottle was taken and transferred to a beaker. 1 mL of ethylene glycol was added and reacted at room temperature for at least 15 minutes. The reaction solution was then titrated with 0.01 mol/L NaOH solution. The titration was terminated when the pH changed abruptly between 6.5 and 8.0. The weight M (unit: g) of NaOH solution consumed during the titration was measured. The density of the 0.01 mol/L NaOH solution is close to that of pure water (the difference is less than 1/1000), so the volume of NaOH solution consumed is also M mL. The degree of crosslinking (D) of dextran microcarriers or dextran was calculated, where D = 1 - (5 × M × 0.01 × 162 / (1000 × 0.2)).

The results are shown in Table 4. The calculated degree of crosslinking of dextran without crosslinking reaction was only 2.86%, which was very close to the proportion of α-(1-3) glycosidic bonds in the dextran molecule (about 3%). With the increase of the amount of crosslinking agent, the degree of crosslinking gradually increased. Within a certain range of crosslinking degree, the microcarrier can be completely dissolved after enzyme treatment. When the degree of crosslinking exceeds a certain range, the microcarrier becomes less likely to be dissolved by enzymes. Based on the results of Example 3, microcarriers with a crosslinking degree between 13.1% and 38.7% can be completely dissolved by enzymes in about half an hour, microcarriers with a crosslinking degree of 44.2% require 1-2 hours to be dissolved by enzymes, and microcarriers with a crosslinking degree of more than 55.1% are difficult to be dissolved by enzymes within 2 hours. The crosslinking degree of commercially available Cytodex 1 microcarriers is 56.7%, meaning only could be partially dissolved by enzymes.

**Table 4. Various dextran microcarriers and their degree of crosslinking**

| Sample name | Dextran | Cr400 | Cr500 | Cr600 | Cr700 | Cr800 | Cr900 | Cr1200 | Cr1500 | Cytode x 1 |
|---|---|---|---|---|---|---|---|---|---|---|
| Degree of crosslinking (D×100% ) | 2.86% | 13.1 % | 19.6 % | 23.3 % | 28.9 % | 38.7 % | 44.2 % | 55.1% | 66.4% | 56.7% |

### Example 5: Cell culture and harvest using a microcarrier

The dextran microcarrier (Cr500) from Example 3 was taken and separated the components between 200-300µm with a standard sieve, 0.4ml of which was suspended in 5mL PBS and autoclaved at 120 degrees for 20 minutes. After sterilization, the microcarriers were washed with alpha MEM medium supplemented with 10% fetal bovine serum, and then 3×10^5 human umbilical cord-derived mesenchymal stem cells (cell passage number P5) were seeded onto the microcarriers and cultured in a cell culture incubator at 37 degrees Celsius and 5% carbon dioxide. On the third day of cultivation, half of the culture medium was removed and an equal amount of fresh culture medium was added. One, three, and six days later, 0.5 mL was taken from the cell culture using microcarriers for cell live and death staining (Elabscience^{®} Calcein AM/PI Double Staining Kit) and microscopic observation. Cell nuclei were released using a solution containing 0.1% crystal violet and 0.1 mol/L citric acid, and cell counts were performed by statistical analysis of the cell nuclei. Six days later, all microcarriers in the culture container with PBS were washed, 5 mL TrypLE and 1% (v/v) glucanase were added, and incubated in a 37°C cell culture incubator for 20-25 minutes to observe the dissolution of the microcarriers. Trypan blue staining and a hemocytometer were used to count the cells after enzyme treatment, and the cell viability and harvest efficiency were calculated. Cell harvest efficiency is the ratio of the number of harvested cells to the number of cells calculated from the cell sampling before harvest. Similarly, Cytodex 1 microcarriers were sterilized and cultured to compare cell proliferation and harvest efficiency.

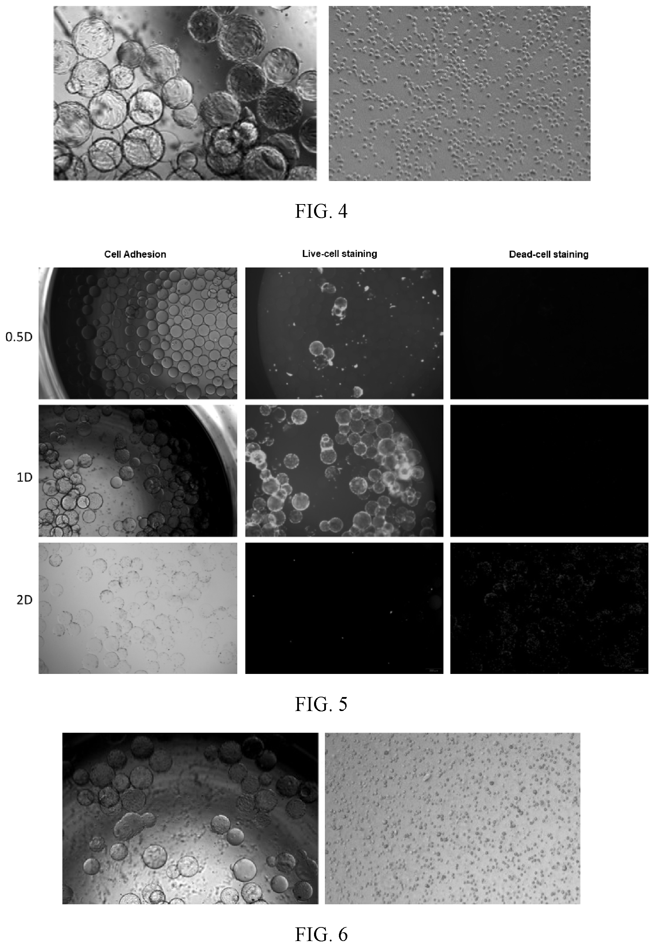

FIG. 3 shows that mesenchymal stem cells adhered well to the Cr500 microcarriers and maintained a high survival rate. After 6 days of culture, the cells expanded approximately 14 times. FIG. 4 shows that the microcarriers were completely dissolved after enzyme treatment, and the cells on their surface were separated. The cell harvest efficiency was as high as 99%, and the cell survival rate was 98%.

After culturing with Cytodex 1 for 6 days, the cells expanded 7.8 times, but the harvest efficiency of cells from Cytodex 1 microcarriers was only 36%.

### Example 6: Positive charge modification of microcarrier surface

According to the steps of Example 3, crosslinked dextran microspheres were prepared using sample Cr500 as the raw material and washed 6 times with pure water. After sieving, six portions of 10 mL crosslinked dextran microspheres were taken, 3 mL of 6M NaOH solution, and 0.1 mL, 0.5 mL, 1 mL, 1.5 mL, 1.75 mL or 2 mL of 1M 2-Dimethylaminoethyl chloride hydrochloride (DEAE) aqueous solution were added to each portion. After mixing, the liquids were stirred in a 60°C water bath for 1 hour. The microcarriers were then washed six times with pure water and twice with PBS to obtain microcarriers with positively charged surfaces. The microcarriers were dried and the degree of crosslinking was measured with the method described in Example 4. The results showed that the degree of crosslinking of the modified microcarrier ranged from 19% to 22%.

**Table 5. Six types of microcarriers with positively charged surfaces**

| Sample name | 0.1D | 0.5D | 1D | 1.5D | 1.75D | 2D |
|---|---|---|---|---|---|---|
| DEAE addition amount (mL) | 0.1 | 0.5 | 1 | 1.5 | 1.75 | 2 |

0.2 mL of each of the above six microcarriers was taken, suspended in 5 mL of PBS, autoclaved, washed twice with the above cell culture medium, and added with 5 mL of culture medium. 3 × 10^5 human umbilical cord-derived mesenchymal stem cells (cell passage number P5) were seeded onto each microcarrier and cultured in a cell culture incubator at 37 degrees Celsius and 5% carbon dioxide. One day and six days later, 0.5 mL was taken from the cell culture using microcarriers for cell live and death staining, microscopic observation, and cell counting. Then 2 mL TrypLE and 1% (v/v) glucanase were added, and incubated in a 37°C cell culture incubator for 20-25 minutes to observe the dissolution of the microcarriers.

FIG. 5 shows that when the positive charge on the surface of the 0.5D microcarrier is too low, cells do not easily adhere to the microcarrier. As the positive charge increases, cells adhere to the microcarrier more easily (sample 1D). However, microcarriers with too much positive charge (sample 2D) can reduce cell survival. FIG. 6 shows that the positively charged microcarriers can be completely dissolved by enzymes, releasing the cells on their surface. The harvest efficiency of cells harvested from microcarriers was over 96%.

### Example 7 Positively charged microcarriers with different degrees of crosslinking

The dextran microspheres (Cr400, Cr500, Cr600, Cr700, Cr800 and Cr900) with different crosslinking degrees without positive charge modification obtained from Example 3 were screened, and six portions of 10mL crosslinked dextran microspheres were taken respectively. 3 mL of 6M NaOH solution and 1 mL of 1M 2-Dimethylaminoethyl chloride hydrochloride (DEAE) aqueous solution was added to each portion. After mixing, the liquids were stirred in a 60°C water bath for 1 hour. The microcarriers were then washed six times with pure water and twice with PBS to obtain microcarriers with different degrees of crosslinking on their surfaces modified by positive charge. The microcarriers were dried and the degree of crosslinking was measured with the method described in Example 4. The results showed that the degree of crosslinking of the modified microcarrier ranged from 13.6% to 44.3%.

According to the method of cell culture and harvest in Example 5, cells were expanded on positively charged microcarriers with different crosslinking degrees. During cell harvest, the microcarriers corresponding to Cr400, Cr500, Cr600, Cr700, and Cr800 were dissolved by enzymes within 30 minutes, while the microcarriers corresponding to Cr900 were dissolved by enzymes within 1-2 hours. Cell harvest efficiency was all above 95%.

### Example 8: Microcarriers with surfaces modified by cell adhesion proteins

According to the steps of Example 3, crosslinked dextran microspheres were prepared using sample Cr500 as the raw material and washed 6 times with pure water. Two portions of 2 mL crosslinked dextran microspheres were taken, 1 mL of 2% recombinant gelatin solution or 1 mL of 25 µg/mL recombinant vitronectin was added, 1 mL of aqueous solution containing 50 mM EDC and 20 mM NHS was added, and the mixture was reacted at room temperature with stirring for 6 hours. After washing with pure water for 6 times, two kinds of microcarriers whose surfaces were modified by cell adhesion proteins were obtained. The microcarriers were dried and the degree of crosslinking was measured with the method described in Example 4. The results showed that the crosslinking degrees of the microcarriers modified with recombinant gelatin or recombinant vitronectin were 19.7% and 20.1%, respectively.

According to the cell culture method described in Example 5, mesenchymal stem cells were cultured for 6 days using more than 0.2 mL of microcarrier. 2 mL of TrypLE solution and 1% (v/v) of glucanase solution were added, and incubated at 37°C for 20 minutes. After the microcarriers are completely dissolved, the cell harvest rates from the recombinant gelatin-modified or recombinant Vitronectin-modified microcarriers are 97% and 99%, respectively, and the cell viability rates are 98% and 99%, respectively.

### Example 9: Preparation of Surface Porous Microcarrier

According to the steps of Example 3, dextran microcarriers were prepared using the raw materials of sample Cr500. After washing the microcarrier with pure water, placed in an ultra-low temperature freezer at -80 degrees Celsius for 12 hours. The frozen microcarriers were freeze-dried at -50 degrees Celsius and 20 Pa for 48 hours using a freeze dryer. A small amount of the microcarrier was then taken and soaked in PBS for observation.

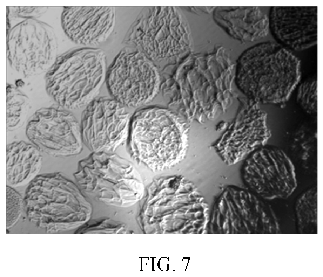

It is observed with an optical microscope that the surface of microcarrier has a pore structure with a pore size of 10-80µm (FIG. 7). The microcarriers were fully dried and the degree of crosslinking was measured with the method described in Example 4. The results showed that the degree of crosslinking of the porous microcarriers on the surface was 19.4% .

### Example 10: Preparation of Pectin Microcarriers

Pectin was dissolved in 30 mL of 6 mol/L sodium hydroxide solution to obtain a 15% pectin solution by mass. 10g pectin solution was taken, 10mL soybean oil and 200µL polyglycerol polyricinoleate (PGPR) were added, and then 500µL epichlorohydrin crosslinking agent was added. Emulsification, crosslinking, and material washing were performed according to the steps in Example 3 to obtain crosslinked pectin microcarriers. The microcarriers were dried and the degree of crosslinking was measured with the method described in Example 4. The degree of crosslinking (D) of the microcarrier was calculated using the following formula: D = 1 - (5 × M × 0.01 × 176 / (1000 × 0.2)) , where M is the volume (mL) of 0.01 M NaOH consumed in the acid-base titration, 176 is the molecular weight of the monosaccharide in the pectin, and 0.2 is the mass (g) of the dried pectin microcarrier. The results showed that the crosslinking degree of the pectin microcarriers was 21.6%, respectively.

According to the cell culture method described in Example 5, mesenchymal stem cells were cultured for 6 days using more than 0.2 mL of microcarrier. 2 mL of TrypLE solution and 1% pectinase solution were added. After incubated at 37 degrees Celsius for 20 minutes, the microcarriers were completely dissolved, the cell harvest rate was 95%, and the cell viability was 96%.

### Example 11: Culture of 293T cells using microcarriers

The dextran microcarriers (Cr600) were sieved and sterilized according to Example 5. After sterilization, the microcarriers were washed with DMEM medium supplemented with 10% fetal bovine serum, and then 3×10^5 293T cells were seeded onto the microcarriers and cultured in a cell culture incubator at 37 degrees Celsius and 5% carbon dioxide. One, three, and six days later, 0.5 mL was taken from the cell culture using microcarriers for cell live and death staining and microscopic observation. On the third day of cultivation, half of the culture medium was removed and an equal amount of fresh culture medium was added. After six days of culture, cells were sampled and counted. Microcarriers were dissolved using TrypLE and 1% volume fraction of glucanase, cells were harvested, and cell viability and harvest efficiency were calculated. Microscopic observation showed that 293T cells adhered well to the microcarriers, with a cell survival rate of over 90%, and the cell harvest rate was 95% after dissolving the microcarriers with enzymes.

Although the present invention has been described according to the specific embodiments above, the inventive concept of the present invention is not limited to this invention. Any modifications that utilize the inventive concept will be included within the scope of protection of this patent.

The above description is only a preferred embodiment of the present invention. The scope of protection of the present invention is not limited to the above embodiments. All technical solutions that fall within the scope of the present invention are within the scope of protection of the present invention. It should be pointed out that for ordinary technicians in this technical field, several improvements and modifications without departing from the principles of the present invention should be also considered to be within the scope of protection of the present invention.

## Claims

1. A microcarrier, **characterized in that**, the microcarrier is a crosslinked microparticle obtained by crosslinking polysaccharide molecules, the microcarrier is able to maintain a stability of a structure of a polysaccharide microcarrier in a cell culture environment, and the microcarrier is able to be completely dissolved by enzyme.

2. The microcarrier according to claim 1, **characterized in that**, the microcarrier is completely dissolved by enzyme within 2 hours.

3. The microcarrier according to claim 1, **characterized in that**, a degree of crosslinking of the polysaccharide molecules in the microcarrier is 13.1%-44.2%.

4. The microcarrier according to claim 1, **characterized in that**, the crosslinked microparticle is a crosslinked microsphere or a quasi-spherical crosslinked microparticle.

5. The microcarrier according to claim 4, **characterized in that**, the microcarrier is able to maintain the stability of a microsphere or quasi-spherical microparticle for more than three days in a normal cell culture environment.

6. The microcarrier according to claim 5, **characterized in that**, the microcarrier is able to maintain the stability of the microsphere or quasi-spherical structure of the polysaccharide microcarrier for more than three days in a cell culture environment of 25-41 °C.

7. The microcarrier according to claim 3, **characterized in that**, the degree of crosslinking is a proportion of a number of monosaccharide residues that crosslink with a crosslinking agent or monosaccharide residues among monosaccharide residues that make up polysaccharide to a number of all monosaccharide residues.

8. The microcarrier according to claim 2, **characterized in that**, the microcarrier is completely dissolved by enzyme within 30 minutes.

9. The microcarrier according to claim 8, **characterized in that**, a degree of crosslinking of the polysaccharide molecules in the microcarrier is 13.1%-38.7%.

10. The microcarrier according to claim 1, **characterized in that**, a surface of the microcarrier is modified.

11. The microcarrier according to claim 10, **characterized in that**, a surface modification of the microcarrier is one or more of positively charged groups or molecules bonded by chemical bonds on the surface, or positively charged groups or molecules physically adsorbed, or proteins, polypeptides or polysaccharides promoting cell adhesion through chemical bonding or physical adsorption.

12. The microcarrier according to claim 10, **characterized in that**, the degree of crosslinking of the polysaccharide molecules in the surface-modified microcarrier is 13.6-44.3%.

13. The microcarrier according to claim 11, **characterized in that**, the physically adsorbed positively charged groups or molecules comprise cationic polymers in diethylaminoethyl dextran, poly -L- ornithine (PLO), poly -D- ornithine (PDO), poly -DL- ornithine, poly -D- lysine (PDL), poly -L- lysine (PLL), poly- DL- lysine, poly -L- arginine (PLA), poly -D- arginine (PDA), poly -DL -arginine, poly -L- homoarginine (PLHA), poly -D- homoarginine (PDHA), poly -DL-homoarginine, poly -L- histidine (PLH), poly -D -histidine (PDH), poly -DL-histidine, polymethylene -CO- guanidine (PMCG), polyallylamine (PAA), polyvinylamine (PVA), polyethyleneimine, allylamine-diallylamine copolymer or allylamine-maleic acid copolymer.

14. The microcarrier according to claim 11, **characterized in that**, the proteins or polypeptides promoting cell adhesion comprises collagen, gelatin, laminin, fibronectin, vitronectin, fragments of collagen, gelatin, laminin, fibronectin or vitronectin promoting cell adhesion, and polypeptides containing arginylglycylaspartic acid.

15. The microcarrier according to claim 1, **characterized in that**, the polysaccharide comprises one or more of following substances: starch, cellulose, dextran, sodium alginate, chitosan, hyaluronic acid, agarose, polygalacturonic acid and pectin, and derivatives of starch, cellulose, dextran, sodium alginate, chitosan, hyaluronic acid, agarose, polygalacturonic acid and pectin.

16. The microcarrier according to claim 1, **characterized in that**, the crosslinking is one or more of chemical crosslinking, photo crosslinking, dehydrogenation thermal crosslinking, and ionic crosslinking.

17. The microcarrier according to claim 16, **characterized in that**, the crosslinking agent used for crosslinking or fixing a chain structure of the polysaccharide molecules in the chemical crosslinking comprise: 1,3- dichloropropanol, N,N' -methylenebisacrylamide, 2,3- dibromo -1-propanol, 1,2,7,8- diepoxyoctane, divinyl sulfone, glutaraldehyde, 1,4- butanediol diglycidyl ether, 1- ethyl- (3- dimethylaminopropyl) carbodiimide hydrochloride, N- hydroxysuccinimide, epichlorohydrin, tannic acid, or genipin.

18. The microcarrier according to claim 16, **characterized in that**, the photo crosslinking is carried out by a light source that is able to initiate a reaction between the crosslinking agent and the polysaccharide molecules.

19. The microcarrier according to claim 16, **characterized in that**, a dehydrogenation thermal crosslinking is carried out in a near-vacuum environment and at high temperature.

20. The microcarrier according to claim 1, **characterized in that**, a surface of the microcarrier is a smooth or porous structure.

21. The microcarrier according to claim 20, **characterized in that**, a pore size of the porous structure is 1-100µm.

22. The microcarrier according to claim 21, **characterized in that**, conditions for controlling the aperture of the porous structure comprise freezing temperature and freezing rate.

23. A preparation method for the microcarrier according to claim 1, **characterized in that**, comprising following steps:
forming a water-in-oil emulsion with a polysaccharide solution, organic solvent which is immiscible with water and emulsifier liquid, adding the crosslinking agent to make the polysaccharide undergo a crosslink reaction; or
adding the crosslinking agent to a polysaccharide solution at first to make polysaccharide undergo a crosslink reaction to obtain a crosslinked polysaccharide solution, and then forming a water-in-oil emulsion with the crosslinked polysaccharide solution, organic solvent which is immiscible with water, and emulsifier liquid.

24. The preparation method according to claim 23, **characterized in that**, a concentration of the polysaccharide in the polysaccharide solution is 10%-50% w/w.

25. The preparation method according to claim 23, **characterized in that**, the method further comprises adding a positively charged substance or a substance promoting cell adhesion after adding the crosslinking agent or at the same time as adding the crosslinking agent, so that a surface of polysaccharide microspheres is positively charged or contains cell adhesion molecules through physical adsorption or chemical reaction.

26. The preparation method according to claim 23, **characterized in that**, the organic solvent which is immiscible with water is vegetable oil.

27. The preparation method according to claim 23, **characterized in that**, an emulsifier in the emulsifier liquid comprises lecithin, carrageenan, guar gum, xanthan gum, polysorbate, cellulose, fatty acid monoglycerides and diglycerides, sucrose esters and sucrose glycerides, fatty acid polyglycerides, polyglycerol polyricinoleate, stearoyl lactate, or dehydrated sorbitol ester.

28. The preparation method according to claim 27, **characterized in that**, the polyglycerol polyricinoleate has a volume fraction of 1-10% in the organic solvent.

29. The preparation method according to claim 23, **characterized in that**, the crosslinking agent is epichlorohydrin.

30. The preparation method according to claim 29, **characterized in that**, a molar ratio of epichlorohydrin to the monosaccharide residues in the polysaccharide is 0.23-0.53:1.

31. The preparation method according to claim 23, **characterized in that**, time of the crosslink reaction is more than 20 hours.

32. A use of the microcarrier according to any one of claims 1-22 as a material for adherent cell culture.

33. The use according to claim 32, **characterized in that** the microcarrier is able to be completely dissolved by enzyme under conditions of 10-39 °C and pH 6.5-8.0.

34. A method for cell adherent culture and cell harvest using the microcarrier according to any one of claims 1-22, **characterized in that**, the method comprises following steps: mixing the microcarrier, culture medium and adherent cells, performing cell culture, then washing the microcarrier, simultaneously or stepwise adding polysaccharide enzyme and enzyme that decomposes proteins or peptides to dissolve the microcarrier, confirming that the microcarrier is completely dissolved, and harvesting the adherent cells.
